# EUROPEAN PATENT APPLICATION

(11) **EP 1 938 751 A1**
(43) Date of publication of application: **02.07.2008**
(21) Application number: 06810706.9
(22) Date of filing: 28.09.2006
(51) Int. Cl.: A61B 6/00

(54) **X-RAY PHOTOGRAPHY APPARATUS**

(30) Priority: 18.10.2005 JP 2005302831
(71) Applicant: Konica Minolta Medical & Graphic, Inc., Hino-shi, Tokyo 191-8511 (JP)
(72) Inventor: YAMANAKA, Kenji, Tokyo 163-0512 (JP)
(74) Representative: Henkel, Feiler & Hänzel
(86) International application number: PCT/JP2006/319248
(87) International publication number: WO 2007/046220

(57) **Abstract**

It is intended to provide an X-ray photography apparatus whereby lungs can be photographed in the desirable inherent size thereof.

An X-ray photography apparatus, having: an X-ray radiation section for radiating an X-ray; an imaging section for detecting the X-ray having passed through an examinee; a memory section for storing maximum inspiration amount data of the examinee; an aspiration flow amount measuring section for measuring an aspiration flow amount of the examinee; and a control section for conducting data processing based on the maximum inspiration amount data of the examinee stored in the memory section and the aspiration flow amount of the examinee measured by the aspiration flow amount measuring section.

## Description

### FIELD OF THE INVENTION

The present invention relates to an X-ray photography apparatus.

### PRIOR ART

In X-ray photography, an X-ray photography technician announces "Take a big breath" and "Hold the breath" to an examinee before taking X-ray photography. However, in practices, there are cases where the examinee does not hole the breath or hole the breath in wrong timing in accordance with announcement, as a result, an X-ray is radiated while the examinee is moving. Thus a problem that desired quality of image cannot be obtained since a blurring is subject to occur.

Now, there is know an X-ray photography apparatus which carries out X-ray photography when the examinee takes breath and hold it using a detector to detect a state of breath of the examinee (for example, Patent Document 1 to 3).

Unexamined Japanese Patent Application Publication No. 2004-57793

Unexamined Japanese Patent Application Publication No. 2001-120528

Unexamined Japanese Patent Application Publication No. H10-43160

### DISCLOSURE OF THE INVENTION

### PROBLEMS TO BE SOLVED BY THE INVENTION

However, in the apparatuses in the above Patent Documents, since photographing is carried out when the examinee take the breath and hold it, the photographing may be carried out when the examinee stops breath though he can breathe further or when the lung is excessively expanded due to excessive inspiration of the examinee, where the lung is not in a preferable inherent size. In these cases, photographing has to be carried out again. If the lung is excessively expanded, a mild atelectasis can be overlooked.

The present invention is achieved to resolve the above problem and an object of the present invention is to provide an X-ray photography apparatus capable of photographing the lung in an inherent preferable state and size.

### MEANS TO SOLVE THE PROBLEMS

The X-ray photography apparatus of the present invention is characterized in that an X-ray generation section to generate a X-ray, an imaging section to detect X-ray having passed through the examinee, a recording section to record maximum inspiration amount data of the examinee, an aspiration flow amount measuring section to measure an aspiration flow amount of the examinee and a control section to conduct processing based on the maximum inspiration amount data of the examinee recorded in the recording section and an aspiration flow amount of the examinee measured by the aspiration flow amount measuring section.

### EFFECTS OF THE INVENTION

According to the present invention, since the maximum inspiration amount data of the examinee is recorded and the current inspiration amount is measured by the aspiration flow amount measuring section, for example, by making the examinee to observe a target inspiration amount based on the maximum inspiration amount data, the examinee is encouraged to try aspiration, thus the lung of the examinee becomes a preferable size for photographing the lung of the examinee. Also, for example, by radiating X-ray when a current inspiration amount is judged to be more than the target inspiration amount, photographing of the lung of the examinee can be carried out in the preferable size.

### BRIEF DESCRIPTION OF THE DRAWINGS

Fig. 1 is a schematic configuration diagram of the X-ray photography apparatus related to the present embodiment.
Fig. 2 is a control configuration diagram of the X-ray photography apparatus related to the present embodiment.
Fig. 3 is a configuration diagram showing an example of an aspiration flow amount measuring section 20 related to the present embodiment.
Fig. 4 (a) is a longitudinal cross-sectional view where a tubal substance 21 related to the present embodiment is cut in an axis direction.
Fig. 4 (b) is a lateral cross-sectional view where a tubal substance 21 related to the present embodiment is cut in a radial direction.
Fig. 5 is crossing view when the tubal substance 21 and holder section 22 related to the present embodiment are cut in an axis direction.
Fig. 6 is a control flow chart of the X-ray photography apparatus related to the present embodiment.
Fig. 7 is an exemplary display of a display section of the X-ray photography apparatus related to the present embodiment.

### DESCRIPTION OF SYMBOLS

- 1: Examinee
- 10: X-ray generation section
- 20: Aspiration flow amount measuring section
- 21: Tubal substance
- 22: Holder section
- 23: Movable member
- 24: CCD area sensor
- 30: Display section
- 40: Apparatus main body
- 41: CPU
- 45: Hard disk (HD)

### PREFERABLE EMBODIMENT OF THE INVENTION

### (Configuration of the apparatus)

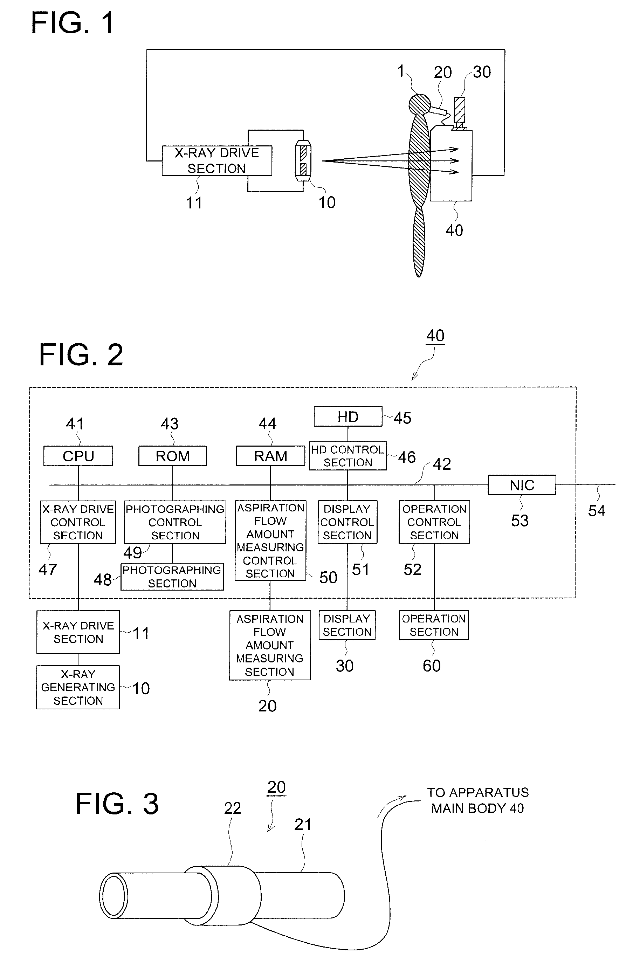

Fig. 1 is a schematic diagram of an X-ray photography apparatus related to the embodiment. An X-ray driving section 11 to drive an X-ray generation section 10 to generate an X-ray for radiating an examinee 1, an aspiration flow amount measuring section 20 to measure an aspiration flow amount of the examinee 1 and a display section 30 arranged to be observed by the examinee 1 are commented to an apparatus main body 40 as the figure shows. Other than the above, an unillustrated operation section (an operation section 60 in Fig. 2 described later) which an X-ray technician operates is connected.

Fig. 2 is a control configuration diagram of the X-ray photography apparatus related to the present embodiment. In side the apparatus main body 40, centering around a CPU 41 representing a control section of the present invention to control operation of the X-ray photography apparatus in accordance with a program, the following sections are connected each other via bus 42.

ROM 43 is a memory to store programs and data necessary for conducting control in the CPU 41 in advance and a RAM 44 is a memory where programs and data necessary for executing control in the CPU 41 are loaded temporary.

A hard disk (HD) 45 representing a memory section of the present invention, where the maximum inspiration amount data of the examinee measured through a publicly know method in advance is matched with identification data to identify the examinee and stored, and image data obtained by X-ray photography is matched with the identification data and stored, is controlled by the CPU 41 via a HD control section 46. Instead of the hard disk, external memory device such as CD-R can be used.

In conjunction with the CPU 41, the X-ray driving control section 47 controls the X-ray driving section 11 and radiation of the X-ray from the X-ray generation section 10.

An imaging section 48 configured with detection elements arranged two dimensionally in a shape of matrix, to detect X-ray having passed through the examinee 1 and to output an electric signal in accordance with detected amount of the X-ray is controlled by the CPU 41 via the image control section 49.

An aspiration flow amount measuring control section 50 controls the aspiration amount measuring section 20 in conjunction with the CPU 41.

A display control section 51 controls the display section 30 in conjunction with the CPU 41.

An operation control section 52 controls the operation section 60 in conjunction with the CPU 41.

A network interface card (NIC) 53 represents an interface for connection via a communication circuit 54, through which data communication with external equipment is carried out.

Fig. 3 is a configuration diagram showing an example of aspiration flow amount measuring section 20 related to the present embodiment. The aspiration flow amount measuring section 20 is provided with a tubal substance 21 and a holder section 22. The tubal substance 21 is configured with a transparent resin to form a flow path where aspiration air flows. Also, the tubal substance 21 and the holder section 22 are configured to be detachable. The holder section 22 is connected to the apparatus main body 40 (refer to Fig. 1).

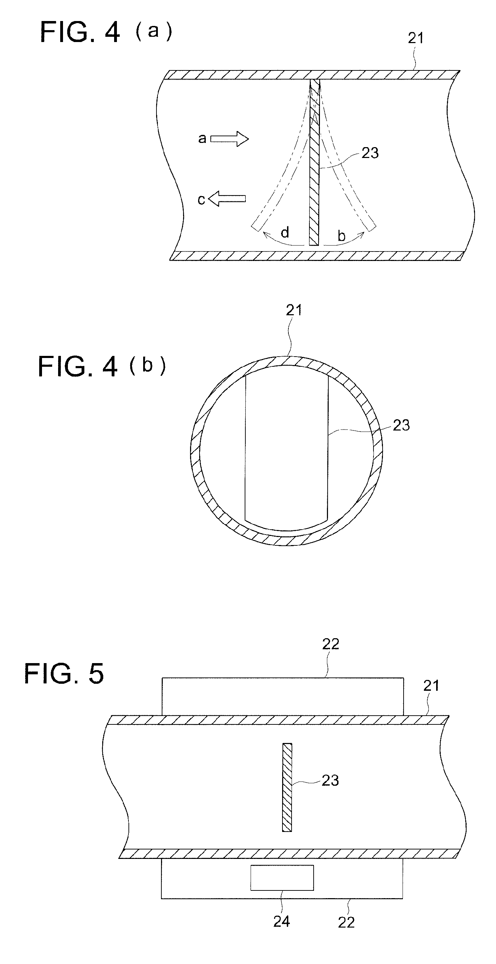

Fig. 4 (a) is a longitudinal cross-sectional view of the tubal substance 21 related to the present embodiment which is cut in an axis direction. As Fig. 4 (a) and Fig. (b) show, in the tubal substance 21, a movable member 23 is disposed in a direction to block the aspiration flow partially and deformed in accordance with a flow amount of the aspiration air.

For example, when the flow amount of exhalation is measured, by blowing exhalation air into the tubal substance 21 an air flow in a direction of an arrow a in Fig. 4 (a) is created. By the flow, the movable member 23 deforms in a direction of an arrow b. When the flow amount of inspiration is measured, by sucking air from the tubal substance 21, an air flow in a direction of an arrow c in Fig. 4 (a) is created. By the flow, the movable member 23 deforms in a direction of an arrow d.

Fig. 5 is a lateral cross-sectional view of the tubal substance 21 and the holder section 22 cut in the axis direction related to the present embodiment. As Fig. 5 shows, at the holder section 22, there is disposed a CCD area sensor 24 representing a detection device of the present invention for photographing the movable member 23 through the transparent tubal substance 21. By detecting deformation of the movable member 23 optically through the CCD area sensor 24, measurement of the aspiration flow amount is possible.

As an apparatus to measure the aspiration flow amount, other than the present embodiment where the aspiration flow amount is measured by detecting the amount of deformation of the movable member 23, a publicly known differential aspiration flow amount measuring apparatus can be used. However, from a view point of superiority in response, it is preferred that the apparatus of the present embodiment where aspiration flow amount is measured by detecting deformation of the movable member 23 is used.

### (A control flow of the apparatus)

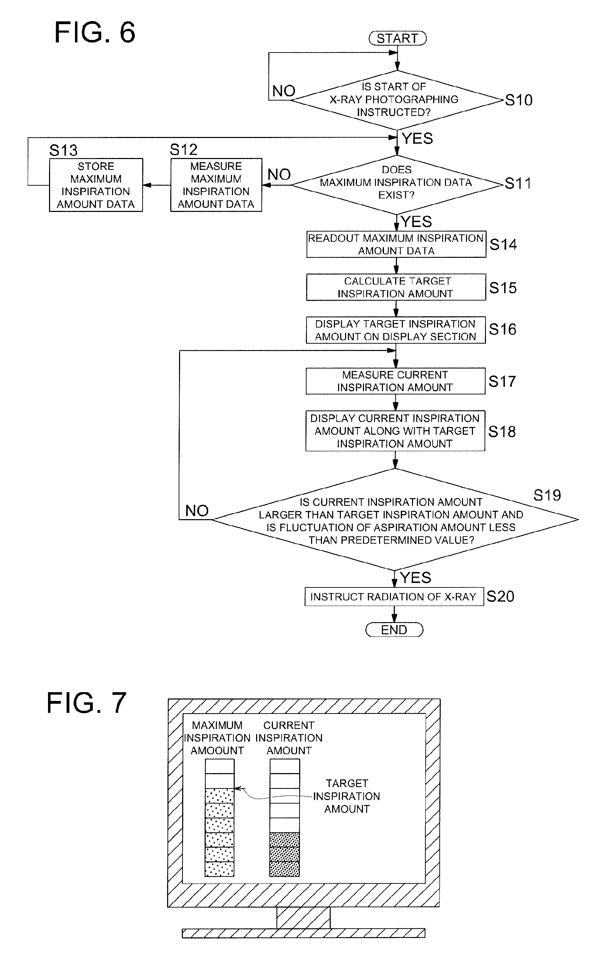

Fig. 6 is a control flow chart of the X-ray photography apparatus related to the present embodiment. The maximum inspiration amount data of the examinee 1 is measured by an apparatus such as a spirometer in advance, and matched with identification data of the examinee to be stored in the HD 45 via a network or via a portable flush memory such as a memory card or an USB memory.

Meanwhile, in case the maximum inspiration amount data of the examinee 1 does not exist at photographing, it is possible to measure the maximum inspiration amount data using the aspiration flow amount measuring section 20 before photographing. However, to obtain an accurate maximum inspiration amount data of the examinee 1, it is preferred that the measuring is carried out under a breath instruction of a nurse.

First, the CPU 41 judges where or not the start of X-ray photography is instructed through the operation control section 52 from the operation section 60 (Step S10). If it is judged that X-ray photography is instructed (Step S10;Yes), the flow proceeds to Step S11, and if it is judged that X-ray photography instruction is not instructed (Step S10;No), the flow returns to the Step S10 and waits for an instruction of start.

In the Step S11, the CPU 41 judges whether or not the maximum inspiration amount data of the examinee exists in the HD 45. If it is judged that the maximum inspiration amount data of the examinee exists in the HD 45 (Step S11; Yes), the flow proceeds to Step S14. If it is judged that the maximum inspiration amount data of the examinee does not exist in the HD 45 (Step S11; No), the examinee 1 performs a maximum inspiration for the aspiration flow amount measuring section 20 and the CPU 41 measures the maximum inspiration amount of the examinee 1 by the aspiration flow amount measuring section 20 through the aspiration flow amount measuring control section 50 (Step S12) and matches the obtained maximum inspiration amount data of the examinee 1 with the identification data and then stores in the HD 45 (Step S13). In order to carry out the maximum inspiration of the examinee 1, the technician may give the aspiration guide to the examinee or the aspiration guide may be given by a screen image or audio through the display section 30.

In the Step S14, the identification data or photographing information (a photographing region, a photographing direction and an X-ray intensity) are inputted through the operation section 60 or an unillustrated hospital information system (HIS), and the CPU 41 reads out the maximum inspiration amount data corresponding to the identification data of the examinee 1 stored in the HD 45.

Next, the CPU 41 calculates the target inspiration amount which the examinee targets when X-ray photography is taken, based on the maximum inspiration data read out in Step S14 and displays the result on the display section 30 via the display control section 51 (Step S16). For example, the target inspiration amount is set at 60 % of the maximum inspiration amount. Thereby observing the display section, the examinee 1 can confirm his own target inspiration amount.

Next, the CPU 41 measures the current inspiration amount of the examinee 1 obtained from the aspiration flow amount measuring section 20 through the aspiration flow amount measuring control section 50 by the examinee 1 to take a breath in accordance with an instruction of the X-ray photography technician (Step S17), and displays a result along with the target inspiration amount on the display section 30 through the display control section 51 (Step S18), for example, as Fig. 7. Thereby the examinee 1 can visually confirm in which level the current inspiration is in respect to the target inspiration amount. If the target inspiration amount is not achieved, the examinee tries taking breath further so as to achieve the target. As the result, the lung of the examinee 1 becomes a preferable size for photographing.

Next, the CPU 41 judges whether or not the current inspiration amount of the examinee 1 obtained from the aspiration flow amount measuring section 20 through the aspiration flow amount measuring control section 50 is more than the target inspiration amount and the aspiration flow amount is not fluctuated (breath is ceased)(Step S19). If it is judged that the current inspiration amount of the examinee 1 obtained is not less than the target inspiration amount, and the fluctuation of the aspiration flow amount is not more than a predetermined value (Step S19; Yes), the CPU 41 instructs the X-ray drive section 11 to radiate the X-ray via the X-ray drive control section 47 and conducts X-ray radiation from the X-ray generation section 10 (Step S20). Thereby, the lung of the examinee 1 is photographed in a state where the lung is in a preferable size. X-ray radiated in Step S20 passes though the examinee 1 and reaches to the imaging section 48, and then the imaging section 48 outputs an electric signal according to the detected X-ray amount. After predetermined processing, the signal thereof is matched with identification data of the examinee 1 as the X-ray image of the examinee 1 and outputted to the HD 45 or the unillustrated HIS and then the processing is terminated.

If it is judged than the current inspiration amount is smaller than the target inspiration amount or a fluctuation of aspiration amount is larger than the predetermined value (Step S19:No), the CPU 41 returns to Step S17, and repeats Step S17 and Step S18 until it is judged that the current inspiration amount is more than the target inspiration value and the fluctuation of the aspiration flow amount is not more than the predetermined value.

In the present embodiment, both are carried out that the current inspiration amount and the target inspiration amount are displayed on the display section 30 to encourage the examinee 1 to try breath, and the X-ray is radiated when the current inspiration amount is not less than the target inspiration amount and the fluctuation of aspiration amount is not more than the predetermined value. Meanwhile, the above case is a more preferable case and the object of the present can be also achieved when either of them is carried out.

For example, the X-ray technician can manually radiate the X-ray after encouraging the examinee 1 to try inspiration by displaying the current inspiration amount and the target inspiration amount on the display 30. Also, for example, encouraging the examinee to try inspiration by guidance of the technician, the X-ray can be radiated when the current inspiration amount exceeds the target inspiration amount and the fluctuation of the inspiration amount is not more than the predetermined value. In this case, a certain advantage can be obtained even if the X-ray is radiated under a condition that the current inspiration amount is simply not less than the target inspiration amount since the size of lung is in a preferable condition.

In the present embodiment, while X-ray radiation is conducted when the current inspiration amount exceeds the target inspiration amount and the fluctuation of the inspiration flow amount is not more than the predetermined value, as mentioned above, if the lung expands excessively, a mild atelectasis may be overlooked, therefore an upper limit of inspiration amount may be provided further and shown on the display section 30 so that X-ray radiation is not instructed if the inspiration amount exceeds the upper limit of inspiration amount.

In the present embodiment, while the display section 30 is disposed so that the examinee 1 can observe, for example, the display section 30 may be disposed so that the X-ray technician can observe. The technician may conduct the guidance such as "take more breath" for the examinee 1, observing the current inspiration amount and the target inspiration amount.

As above, according to the present invention, the maximum inspiration amount data of the examinee 1 is stored in the HD45 and the current inspiration amount is measure by the aspiration flow amount measuring section 20, thereby, for example, by calculating the target inspiration amount from the maximum inspiration amount data and by the examinee 1 to observe the target inspiration amount and the current inspiration amount, the examinee 1 is encouraged to try inspiration thus the condition of the lung of the examinee 1 can be a preferable size. Also, for example, by radiating the X-ray when the current inspiration amount is judged to be not less than the target inspiration amount, the lung of the examinee 1 can be photographed in a condition where the lung is a preferable size.

## Claims

1. An X-ray photography apparatus, comprising:
an X-ray generation section for radiating an X-ray;
an imaging section for detecting the X-ray having passed through an examinee;
a memory section for storing maximum inspiration amount data of the examinee;
an aspiration flow amount measuring section for measuring an aspiration flow amount of the examinee; and
a control section for conducting data processing based on the maximum inspiration amount data of the examinee stored in the memory section and the aspiration flow amount of the examinee measured by the aspiration flow amount measuring section.

2. The X-ray photographing apparatus of claim 1, further comprising:
a display section;
wherein the control section displays a target inspiration amount based on the maximum inspiration amount data of the examinee stored in the memory section and the aspiration flow amount of the examinee measured by the aspiration flow amount measuring section on the display section.

3. The X-ray photographing apparatus of any one of claims 1 or 2, wherein the control section allows radiation of the X-ray by the X-ray generation section in case the aspiration flow amount of the examinee measured by the aspiration flow amount measuring section is not less than the target inspiration amount based on the maximum inspiration amount data stored in the memory section.

4. The X-ray photographing apparatus of claim 3, wherein the control section allows radiation of the X-ray by the X-ray generation section in case a fluctuation of the aspiration flow amount of the examinee measured by the aspiration flow amount measuring section is not more than a predetermined value.

5. The X-ray photographing apparatus of claim 2, wherein an inspiration amount upper limit based on the maximum inspiration amount data of the examinee stored in the memory section is displayed on the display section.

6. The X-ray photographing apparatus of claim 5, wherein the control section allows radiation of the X-ray by the X-ray generation section in case the aspiration flow amount of the examinee measured by the aspiration flow amount measuring section is not less than the target inspiration amount based on the maximum inspiration amount data stored in the memory section, and is not more than the inspiration amount upper limit based on the maximum inspiration amount data of the examinee stored in the memory section.

7. The X-ray photographing apparatus of any one of claims 1 to 6, further comprising:
a tubal substance in which aspiration air flows;
a movable member, disposed in the tubal substance in a direction to block a flow of the aspiration air partially or totally, which physically changes in accordance with a flow amount of the aspiration air; and
a detection device, disposed outside the tubal substance, for detecting an amount of physical change of the movable member in a non-contact manner.
